# EUROPÄISCHE PATENTANMELDUNG

(11) **EP 0 695 742 A1**
(43) Veröffentlichungstag der Anmeldung: **07.02.1996**
(21) Anmeldenummer: 95111589.8
(22) Anmeldetag: 24.07.1995
(51) Int. Cl.: C07D 203/26, C07D 263/52, H01L 39/12, G02B 1/04

(54) **Fullerenazirin-Derivate, Verfahren zu ihrer Herstellung und deren Verwendung**

(30) Priorität: 03.08.1994 DE 4427489; 15.12.1994 DE 4444684
(71) Anmelder: HOECHST AKTIENGESELLSCHAFT, D-65929 Frankfurt am Main (DE)
(72) Erfinder: Bingel, Carsten, Dr., D-65830 Kriftel (DE); Mattay, Jochen, Prof.Dr., D-48341 Altenberge (DE); Averdung, Johannes, Dl., D-48149 Münster (DE); Abraham, Werner, Prof.Dr., D-16547 Birkenwerder (DE); Jacobi, Dirk, Dl., D-13189 Berlin (DE)

(57) **Zusammenfassung**

Die Erfindung betrifft Fullerenderivate der Formel I 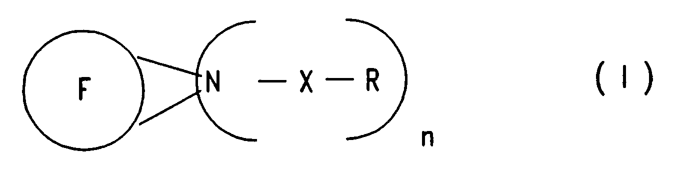
worin die Symbole und Indices folgende Bedeutung haben:
- F:: ein Fullerenrest der Formel C₂₀₊₂ₘ mit m = 2 bis 100;
- X:: C = O, SO₂, SO, P(O)R, P(S)R oder PR;
- R:: eine geradkettige oder verzweigte C₁-C₂₀-Alkylgruppe, eine C₆-C₁₄-Aryl-C₁-C₁₀-alkylgruppe oder eine C₆-C₁₄-Arylgruppe, wobei in der Alkylgruppe eine oder mehrere nicht benachbarte CH₂-Gruppen durch -C ≡ C-, -CH = CH-, -O-, -S-, -COO-, -SiR₂- und/oder -CO- ersetzt sein können, Alkyl und Aryl unabhängig voneinander ein- oder mehrfach gleich oder verschieden durch OH, OR¹, COOR, OCOR, F, Cl, Br, NO₂, CN, NHCOR¹ oder NR¹COR¹ sowie Aryl durch R¹ substituiert sein können und R¹ eine C₁-C₆-Alkylgruppe ist;
- n:: eine natürliche Zahl von 1 bis 10 + m mit m = 2 bis 100;
sowie Fullerenoxazole, die beispielsweise durch Umlagerung der Fullerenderivate der Formel I erhalten werden;
Verfahren zu deren Herstellung und ihre Verwendung.

## Beschreibung

Fullerene sind käfigförmige Kohlenstoffallotrope der allgemeinen Formel (C₂₀₊₂ₘ), wobei m eine natürliche Zahl ist. Sie enthalten zwölf Fünf- sowie beliebig viele, mindestens aber zwei Sechsringe aus Kohlenstoffatomen. Obwohl diese Verbindungsklasse erst 1985 von Kroto und Smalley nachgewiesen wurde (Nature, 1985, 318, 162) und Krätschmer und Huffman erstmals 1990 über die Darstellung makroskopischer Mengen an C₆₀ berichteten (Nature 1990, 347, 354), sind solche Verbindungen sehr schnell auf ein breites Interesse gestoßen und wurden innerhalb kürzester Zeit Gegenstand zahlreicher Forschungsarbeiten (siehe z.B. G.S. Hammond, V.J. Kuck (Editors), Fullerenes, American Chemical Society, Washington DC 1992 und Accounts of Chemical Research, Märzausgabe 1992).

Da man ein hohes Potential dieser Stoffklasse, beispielsweise im Bereich der Optoelektronik und der Wirkstofforschung erwartet, wurden bereits umfangreiche Untersuchungen zur gezielten Derivatisierung, insbesondere von C₆₀ und C₇₀, unternommen (siehe z.B. R. Taylor, D.R.M. Walton, Nature 1993, 363, 685 und A. Hirsch, Angew. Chem. 1993, 105, 1189). In verschiedenen Derivatisierungsversuchen gelang es, definierte Monoaddukte von C₆₀ zu isolieren.

Cyclopropanderivate wurden beispielsweise durch die Umsetzung von Fullerenen in 1,3 dipolaren Cycloadditionen mit Diazomethanderivaten unter Stickstoffeliminierung (siehe z.B. F. Wudl et al., Acc. Chem. Res. 1992, 25, 157 und F. Diederich et al., Helv. Chem. Acta 1993, 76, 1231), in [2 + 1] Carbenadditionen mit nucleophilen Glycosylidencarbenen (siehe z.B. A. Vasella et al., Angew. Chem. 1992, 104, 1383) und durch Umsetzung mit stabilisierten α-Halocarbanionen (z.B. C. Bingel, Chem. Ber. 1993, 126, 1957) erhalten.

Erste Beispiele für die den Cyclopropanderivaten entsprechenden Stickstoffheterocyclen wurden beschrieben. So erhält man bei der thermisch aktivierten Umsetzung von C₆₀ mit Benzyl- und Alkoxymethylen-aziden 5-6 Ring Azafulleroide (M. Prato, Q. Chan Li, F. Wudl, J. Am. Chem. Soc. 1993, 115, 1148). 6-6-Ring Fullerenaziridine mit Carbamatstruktur wurden durch die thermisch aktivierte Umsetzung von C₆₀ mit Azidoameisensäureestern dargestellt (T. Ishida, K. Tanaka, T. Nogami, Chem. Lett. 1994, 561; N.R. Banks et al., J. Chem. Soc., Chem. Commun. 1994, 1365).

Während Ishida et al. das von ihnen hergestellte 6-6-Ring Fullerenaziridin-Derivat mit Carbamatstruktur nach 8 Stunden in siedendem ortho-Dichlorbenzol ohne Veränderungen zurückerhalten, beschreiben Banks et al., daß das von ihnen untersuchte 6-6-Ring Fullerenaziridin mit Carbamatstruktur durch Refluxieren in Tetrachlorethan zu einem Fulleren-4,5-oxazol isomerisiert.

Aufgabe der vorliegenden Erfindung ist es, definierte Fullerenderivate zu synthetisieren, die Struktureinheiten mit solchen funktionellen Gruppen enthalten, wodurch sich einerseits die physikalischen Eigenschaften wie Löslichkeit und Polarität verbessern und wodurch andererseits weitere chemische Umsetzungen zu neuen Fullerenderivaten ermöglicht werden.

Es wurde überraschend gefunden, daß sich Fullerenaziridine mit Säureamidfunktion beispielsweise aus Fulleren und Acylnitrenen herstellen lassen, und daß die Umsetzung von N-Halosäure-amidanionen mit Fullerenen ebenfalls Fullerenaziridine liefert.

Gegenstand der Erfindung ist ein Fullerenderivat mit Säureamidfunktion der Formel I 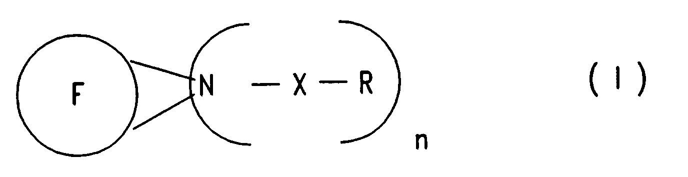
worin die Symbole und Indices folgende Bedeutung haben:
- F:: ein Fullerenrest der Formel C₂₀₊₂ₘ mit m = 2 bis 100;
- X:: C = O, SO₂, SO, P(O)R, P(S)R oder PR;
- R:: eine geradkettige oder verzweigte C₁-C₂₀-Alkylgruppe, eine C₆-C₁₄-Aryl-C₁-C₁₀-alkylgruppe oder eine C₆-C₁₄-Arylgruppe, wobei in der Alkylgruppe eine oder mehrere nicht benachbarte CH₂-Gruppen durch -C ≡ C-, -CH = CH-, -O-, -S-, -COO-, -SiR₂- und/oder -CO- ersetzt sein können, Alkyl und Aryl unabhängig voneinander ein- oder mehrfach gleich oder verschieden durch OH, OR¹, COOR, OCOR, F, Cl, Br, NO₂, CN, NHCOR¹ oder NR¹COR¹ sowie Aryl durch R¹ substituiert sein können und R¹ eine C₁-C₆-Alkylgruppe ist;
- n:: eine natürliche Zahl von 1 bis 10 + m mit m = 2 bis 100.

Bevorzugt sind Verbindungen der Formel I, in der die Symbole und Indices die folgende Bedeutung haben:
- F:: ein Fullerenrest der Formel C₂₀₊₂ₘ mit m = 20, 25, 28 und/oder 29;
- X:: C = O, SO₂ oder SO;
- n:: eine natürliche Zahl von 1 bis 10 + m mit m = 20, 25, 28 und/oder 29 und R die obengenannte Bedeutung hat.

Besonders bevorzugt sind Verbindungen der Formel I, in der die Symbole und Indices die folgende Bedeutung haben:
- F:: C₆₀ und/oder C₇₀;
- X:: C = O oder SO₂;
- R:: eine geradkettige oder verzweigte C₁-C₁₀-Alkylgruppe, eine Phenyl-C₁-C₆-alkylgruppe oder eine Phenylgruppe, wobei Alkyl und Phenyl unabhängig voneinander ein- bis dreifach gleich oder verschieden durch OH, OR¹, COOR, OCOR, F, Cl, Br, NO₂, CN, NHCOR¹ oder NR¹COR¹, sowie Phenyl durch R¹ substituiert sein können und R¹ eine C₁-C₆-Alkylgruppe ist;
- n:: eine natürliche Zahl von 1 bis 6.

Ganz besonders bevorzugt sind Verbindungen der Formel I, in denen die Symbole und Indices folgende Bedeutung haben:
- F:: C₆₀;
- X:: C = O oder SO₂;
- R:: eine geradkettige oder verzweigte C₁-C₆-Alkylgruppe oder eine Phenylgruppe, wobei Alkyl und Phenyl unabhängig voneinander ein- bis dreifach gleich oder verschieden durch OH, OR², COOR², OCOR², F, Cl, Br, NO₂ oder CN sowie Phenyl durch R² substituiert sein können und R² Methyl oder Ethyl ist;
- n:: 1 oder 2.

Die Bildung von Aziridinen aus Olefinen mit verschiedenen Reagenzien, wie die direkte Nitrenaddition an Olefine oder die 1,3-dipolare Cycloaddition von Aziden an Olefine, gefolgt von Zersetzung des intermediären Triazols, ist bekannt (J.E.G. Kemp, Comprehensive Organic Synthesis, Vol. 7, Hrsg.: B.M. Trost, I. Fleming, Pergamon Press, Oxford, 1991, 469).

Weiterer Gegenstand der Erfindung sind zwei Verfahren zur Herstellung von Fullerenderivaten der Formel I.

Im ersten Verfahren wird ein Fulleren der Formel C₂₀₊₂ₘ, worin m = 2 bis 100 ist, mit einem Säureazid der Formel II,

R-X-N₃ (II),

wobei R und X die oben genannten Bedeutungen haben, in einem inerten Lösungsmittel bei einer Temperatur von -78°C bis 180°C zu einer Verbindung der Formel I umgesetzt.

Die Umsetzung erfolgt dabei bevorzugt in Gegenwart einer Lichtquelle, die Licht der Wellenlänge 230 bis 380 nm, bevorzugt 290 bis 310 nm, emittiert.

Zur Darstellung der 6-6-Ring Fullerenaziridinderivate wird beispielsweise in einem chlorierten Lösungsmittel gearbeitet. Als vorteilhaft erweist sich beispielsweise 1,1,2,2-Tetrachlorethan und Temperaturen bis 40°C.

In 1,1,2,2-Tetrachlorethan werden im Vergleich zu Methylenchlorid bei der Reaktion höhere Ausbeuten erhalten. Im Gegensatz dazu werden beispielsweise bei Verwendung von Benzol als Lösungsmittel unter den photochemischen Bedingungen geringe Mengen der 5-6-Ring Azafulleroide gebildet.

Im zweiten Verfahren wird Fulleren der Formel C₂₀₊₂ₘ, worin m = 2 bis 100 ist, mit einem stabilisierten Stickstoffanion der Formel III 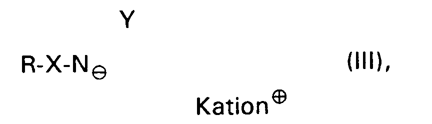
wobei R und X die oben genannten Bedeutungen haben,
- Y:: -Cl, -Br oder -I und
- Kation:: Alkalimetall, Tetraalkyl(aryl)ammonium, Tetraalkyl(aryl)phosphonium oder Hexaalkyl(aryl)guanidinium
bedeuten, in einem aprotischen Lösungsmittel wie zum Beispiel Toluol, Chlorbenzol oder CH₂Cl₂ bei einer Temperatur von -78°C bis 180°C, bevorzugt bei 0°C bis 110°C, besonders bevorzugt bei 20°C bis 30°C, zu einer Verbindung der Formel I umgesetzt.

Bevorzugt werden bei der Herstellung der Verbindungen der Formel I als Ausgangsverbindungen Fullerene der allgemeinen Formel C₂₀₊₂ₘ mit m = 20, 25, 28 und/oder 29, besonders bevorzugt mit m = 20 und/oder 25, eingesetzt.

Die Fullerene können durch Herstellung von Fullerenruß im Lichtbogenverfahren mit anschließender Extraktion mit einem unpolaren organischen Lösungsmittel, wie in WO 92/09279 beschrieben, als Rohfulleren gewonnen werden. Die weitere Feinauftrennung kann säulenchromatographisch erfolgen.

Die eingesetzten Fullerene sind zum Teil auch Handelsprodukte.

Es können aber auch beliebige Fullerenderivate, wie z.B. Monoaddukte oder Polyaddukte, die durch Derivatisierung von Fullerenen zugänglich sind (siehe z.B. R. Tayler, D.R.M. Walton, Nature 1993, 363, 685 und A. Hirsch, Angew. Chem. 1993, 105, 1189) und die unter den hier beschriebenen Reaktionsbedingungen stabil und inert sind, eingesetzt werden.

Die Verbindungen der Formel II sind bekannt. Ihre Synthese erfolgt beispielsweise aus dem entsprechenden Säurechlorid und einem Azid:
- Organikum, 16. bearb. Aufl. 1986, S. 702
- Houben-Weyl, Methoden der Organischen Chemie IX, Schwefel-, Selen-, Tellur-Verbindungen, S. 653
Die Verbindungen der Formel III sind zum Teil Handelsprodukte oder lassen sich nach bekannten Methoden herstellen:
- Organikum, 16. bearb. Aufl. 1986, S. 700
Ferner lassen sich durch thermische Isomerisierung der erfindungsgemäßen 6-6-Ring Fullerenaziridine der Formel I unter anderen 6-6-Ring Fullerenoxazole gewinnen, beispielsweise durch mehrstündiges Refluxieren in 1,1,2,2-Tetrachlorethan oder einfach durch mehrstündiges Erhitzen des Feststoffes, vorzugsweise im Bereich von 140 bis 200°C.

Ein weiterer Gegenstand der vorliegenden Erfindung ist deshalb ein Fullerenderivat der Formel IV 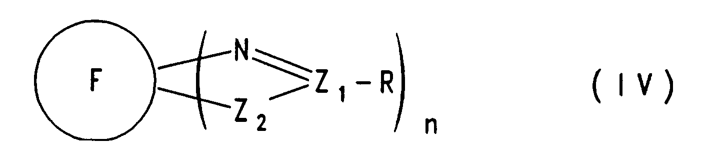
worin die Symbole und Indices folgende Bedeutung haben:
- F:: ein Fullerenrest der Formel C₂₀₊₂ₘ mit m = 2 bis 100;
- Z₁:: C, S, S = O oder P;
- Z₂:: O oder S;
- R:: eine geradkettige oder verzweigte C₁-C₂₀-Alkylgruppe, eine C₆-C₁₄-Aryl-C₁-C₁₀-alkylgruppe oder eine C₆-C₁₄-Arylgruppe, wobei in der Alkylgruppe eine oder mehrere nicht benachbarte CH₂-Gruppen durch -C ≡ C-, -CH = CH-, -O-, -S-, -COO-, -SiR₂- und/oder -CO- ersetzt sein können, Alkyl und Aryl unabhängig voneinander ein- oder mehrfach gleich oder verschieden durch OH, OR¹, COOR, OCOR, F, Cl, Br, NO₂, CN, NHCOR¹ oder NR¹COR¹ sowie Aryl durch R¹ substituiert sein können und R¹ eine C₁-C₆-Alkylgruppe ist;
- n:: eine natürliche Zahl von 1 bis 10 + m mit m = 2 bis 100.

Bevorzugt sind Verbindungen der Formel IV, in der die Symbole und Indices die folgende Bedeutung haben:
- F:: ein Fullerenrest der Formel C₂₀₊₂ₘ mit m = 20, 25, 28 und/oder 29;
- Z₁:: C oder S = O;
- Z₂:: O;
- n:: eine natürliche Zahl von 1 bis 10 + m mit m = 20, 25, 28 und/oder 29 und R die obengenannte Bedeutung hat.

Besonders bevorzugt sind Verbindungen der Formel IV, in der die Symbole und Indices die folgende Bedeutung haben:
- F:: C₆₀ und/oder C₇₀;
- Z₁:: C;
- Z₂:: O;
- R:: eine geradkettige oder verzweigte C₁-C₁₀-Alkylgruppe, eine Phenyl-C₁-C₆-alkylgruppe oder eine Phenylgruppe, wobei Alkyl und Phenyl unabhängig voneinander ein- bis dreifach gleich oder verschieden durch OH, OR¹, COOR, OCOR, F, Cl, Br, NO₂, CN, NHCOR¹ oder NR¹COR¹, sowie Phenyl durch R¹ substituiert sein können und R¹ eine C₁-C₆-Alkylgruppe ist;
- n:: eine natürliche Zahl von 1 bis 6.

Ganz besonders bevorzugt sind Verbindungen der Formel IV, in denen die Symbole und Indices folgende Bedeutung haben:
- F:: C₆₀;
- Z₁:: C;
- Z₂:: O;
- R:: eine geradkettige oder verzweigte C₁-C₆-Alkylgruppe oder eine Phenylgruppe, wobei Alkyl und Phenyl unabhängig voneinander ein-bis dreifach gleich oder verschieden durch OH, OR², COOR², OCOR², F, Cl, Br, NO₂ oder CN sowie Phenyl durch R² substituiert sein können und R² Methyl oder Ethyl ist;
- n:: 1 oder 2.

Die erfindungsgemäßen Verbindungen der Formel I und IV finden beispielsweise Anwendung in optoelektrischen Bauelementen. Die Erfindung wird durch die Beispiele näher erläutert.

### Beispiel 1

Zu einer Lösung von 145 mg (0.20 mmol) C₆₀ in 600 ml Dichlormethan wurden 162 mg (1.10 mmol) Benzoylazid gegeben. Die Lösung wurde in Pyrexglasröhrchen gefüllt, mit Argon gespült und bei λₘₐₓ = 300 nm 60 Minuten im RPR 100 Rayonet Photochemical Chamber Reactor mit RPR 3000-Å Lampen bestrahlt. Dabei änderte sich die Farbe der Lösung von violett nach braunrot. Das Lösungsmittel wurde destillativ im Vakuum bei 40°C entfernt. Nicht umgesetztes C₆₀ wurde säulenchromatographisch (200 g Merck Kieselgel 60, Korngröße 63 bis 200 µm; n-Hexan) abgetrennt, und anschließend wurde eine weinrote Produktfraktion mit Toluol/n-Hexan 50:50 eluiert. Nach Entfernen des Lösungsmittels wurden 30 mg (18 %) der 6,6-verbrückten Verbindung der Formel 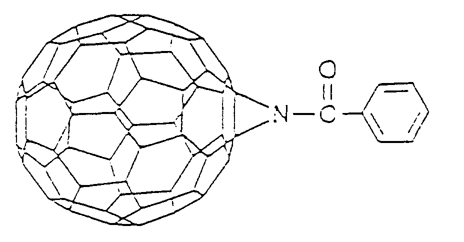
als mikrokristalliner schwarzgrauer Feststoff gewonnen.
MS m/z (%)
- DEI (70 kV):: 839 (6; MH^{·}⁺), 838 (10; M^{·}⁺-H), 721 (46), 720 (100; M^{·}⁺-C₇H₅NO).
¹H-NMR (360 MHz; CS₂/Aceton-d₆ 10:1)
- δ:: 8.48 (m, 2H), 7.73 (m, 1H), 7.64 (m, 2H) ppm.
¹³C-NMR (90.5 MHz; CS₂/Aceton-d₆ 10:1)
- δ:: 169.77, 145.71, 145.61, 145.39, 145.30, 145.08, 145.04, 144.61, 144.32, 144.29, 143.66, 143.63, 143.33, 142.72, 142.64, 141.63, 140.60, 134.40, 131.51, 129.83, 129.72, 81.62 ppm.
FT-IR (KBr, 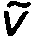 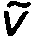 in cm⁻¹⁾
1700 (s, C = O), 1448 (w), 1427 (m, T₁ᵤC₆₀), 1395 (m), 1316 (w), 1264 (s), 1254 (s), 1182 (m, T₁ᵤC₆₀), 1043 (m), 1021 (m), 692 (s), 576 (m, T₁ᵤC₆₀), 527 (ss, T₁ᵤC₆₀)
US-Vis λₘₐₓ in nm (ε in l mol⁻¹cm⁻¹)
- n-Hexan:: (c = 4.3 · 10⁻⁵): 208 (60000), 255 (42000), 322 (11000), 420 (400-500).
- Toluol:: 330, 425, 495.

### Beispiel 2

Das in Beispiel 1 charakterisierte Monoaddukt wurde ebenfalls folgendermaßen hergestellt.

Eine Lösung von 288 mg (0.40 mmol) C₆₀ und 252 mg Benzoylazid in 80 ml 1,1,2,2-Tetrachlorethan wurde 7.5 Stunden analog zu Beispiel 1 bestrahlt. Nach Fällung der Fullerenverbindungen mit Acetonitril, Isolierung und Waschen derselben mit Acetonitril, wurde der Rückstand mit Toluol extrahiert und das Extrakt unter vermindertem Druck eingeengt. Der Rückstand wurde an Kieselgel (Korngröße 63 bis 200 µm) mit n-Hexan und n-Hexan/Toluol 3:2 bis 1:1 chromatographiert. In der ersten Fraktion wurden 63 mg (22 %) C₆₀, in der zweiten Fraktion 133 mg (40 %; 51 % bezogen auf umgesetztes C₆₀) des in Beispiel 1 charakterisierten Monoadduktes und in der dritten Fraktion 55 mg Polyaddukte erhalten.

### Beispiel 3

Zu einer Lösung von 144 mg (0.20 mmol) C₆₀ in 600 ml Dichlormethan wurden 197 mg (0.87 mmol) p-Brombenzoylazid gegeben. Die Lösung wurde in Pyrexglasröhrchen gefüllt, mit Argon gespült und bei λₘₐₓ = 300 nm 60 Minuten im RPR 100 Rayonet Photochemical Chamber Reactor mit RPR 3000-Å Lampen bestrahlt. Dabei änderte sich die Farbe der Lösung von violett nach braunrot. Das Lösungsmittel wurde destillativ im Vakuum bei 40°C entfernt. Nicht umgesetztes C₆₀ wurde säulenchromatographisch (200 g Merck Kieselgel 60, Korngröße 63 bis 200 µm; n-Hexan) abgetrennt, und anschließend wurde eine weinrote Produktfraktion mit Toluol/n-Hexan 50:50 eluiert. Die Produktfraktion wurde auf 5 ml eingeengt, das Addukt mit 30 ml Acetonitril ausgefällt und mit einem Ultrafilter abfiltriert. Nach Trocknen im Vakuum wurden 20 mg (11 %) der 6,6-verbrückten Verbindung der Formel 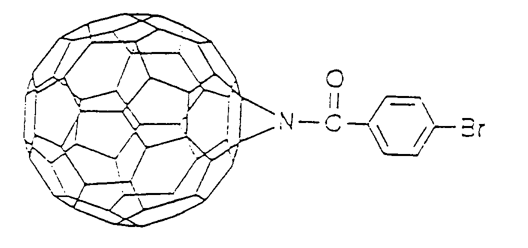
als mikrokristalliner schwarzgrauer Feststoff gewonnen.
MS m/z ≧ 400 (%)
- DEI (70 kV):: 919 (5; MH^{·}⁺), 917 (3; MH^{·}⁺), 721 (73), 720 (100; M^{·}⁺-C₇H₄NOBr)
¹H-NMR (300 MHz, CS₂/Aceton-d₆ 10:1)
- δ:: 8.40 (m,2H), 7.83 (m,2H) ppm.
¹³C-NMR (90.5 MHz; CS₂/Aceton-d₆ 10:1)
- δ:: 169.29, 145.80, 145.70, 145.50, 145.40, 144.63, 144.35, 144.08, 143.73, 143.71, 143.40, 142.77, 142.65, 141.72, 140.67, 133.18, 132.92, 131.80, 131.33, 81.48 ppm.
UV-Vis λₘₐₓ in nm (ε in l mol⁻¹ cm⁻¹)
- n-Hexan:: (c=6.5 · 10⁻⁵): 211 (48000), 255 (43000), 318 (13000), 421 (1300),
- Toluol:: 326, 425, 371

### Beispiel 4

Das in Beispiel 3 charakterisierte Monoaddukt wurde ebenfalls folgendermaßen hergestellt.

Eine Lösung von 293 mg (0.41 mmol) C₆₀ und 368 mg (1.63 mmol) 4-Brombenzoylazid in 80 ml 1,1,2,2-Tetrachlorethan wurde 5 Stunden analog zu Beispiel 3 bestrahlt. Nach Fällung der Fullerenverbindungen mit 600 ml Acetonitril wurde der Feststoff durch Filtration isoliert und mit Acetonitril gewaschen. Der Rückstand wurde an Kieselgel (Korngröße 63 bis 200 µm) mit n-Hexan und n-Hexan/Toluol 3:2 bis 1:1 chromatographiert. In der ersten Fraktion wurden 154 mg (52 %) C₆₀, in der zweiten Fraktion 78 mg (21 %; 44 % bezogen auf umgesetztes C₆₀) des in Beispiel 3 charakterisierten Monoaddukts und in der dritten Fraktion 47 mg Diaddukt erhalten.

### Beispiel 5

Zu einer Lösung von 148 mg (0.21 mmol) C₆₀ in 600 ml Dichlormethan wurden 154 mg (1.03 mmol) p-Methoxybenzoylazid gegeben. Die Lösung wurde in Pyrexglasröhrchen gefüllt, mit Argon gespült und bei λₘₐₓ = 300 nm 60 Minuten im RPR 100 Rayonet Photochemical Chamber Reactor mit RPR 3000-Å Lampen bestrahlt. Dabei änderte sich die Farbe der Lösung von violett nach braunrot. Das Lösungsmittel wurde destillativ im Vakuum bei 40°C entfernt und das Reaktionsgemisch durch Säulenchromatographie (200 g Merck Kieselgel 60, Korngröße 63 bis 200 µm; Toluol/n-Hexan 50:50) getrennt. Nach Entfernen des Lösungsmittel wurden 19 mg (11 %) der 6,6-verbrückten Verbindung der Formel 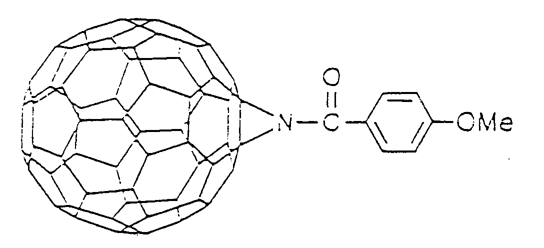
als mikrokristalliner, schwarzgrauer Feststoff gewonnen.
MS m/z ≧ 400 (%)
DEI (70 kV): 869 (24; MH^{·}⁺), 722 (57), 721 (88), 720 (100; M^{·}⁺-C₈H₇NO₂)
¹H-NMR (360 MHz; CS₂/Aceton-d₆ 10:1)
- δ:: 8.44 (m,2H), 7.13 (m,2H), 3.96 (s,3H) ppm.
¹³C-NMR (90.5 MHz; CS₂/Aceton-d₆ 10:1)
- δ:: 169.34, 145.85 145.77, 145.68, 145.42, 145.36, 145.21, 145.11, 144.72, 144.67, 144.37, 143.71, 143.69, 143.41, 142.80, 142.75, 141.67, 140.66, 132.02, 123.76, 115.39, 81.82, 55.89 ppm.
FT-IR (KBr, 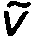 in cm⁻¹)
1693 (s, C = O), 1602 (s), 1508 (m), 1459 (w), 1428 (m, T₁ᵤC₆₀), 1396 (m), 1316 (w), 1265 (m), 1254 (s), 1182 (m, T₁ᵤC₆₀), 1164 (s), 1092 (w), 1043 (m), 1026 (m), 843 (m), 793 (m), 727 (m), 694 (m), 576 (m, T₁ᵤC₆₀), 527 (ss, T₁ᵤC₆₀).
UV-Vis λₘₐₓ in nm (ε in l mol⁻¹ cm⁻¹)
- n-Hexan:: (c = 6.5 · 10⁻⁵): 211 (64000), 257 (54000), 318 (15000), 421 (1200).
- Toluol:: 330, 425, 493

### Beispiel 6

Das in Beispiel 5 charakterisierte Monoaddukt wurde ebenfalls folgendermaßen herstellt.

Eine Lösung von 434 mg (0.60 mmol) C₆₀ und 428 mg (2.42 mmol) 4-Methoxybenzoylazid in 120 ml 1,1,2,2-Tetrachlorethan wurde 5 Stunden analog zu Beispiel 5 bestrahlt und analog zu Beispiel 4 aufgearbeitet. Der Rückstand wurde an Kieselgel (Korngröße 63 bis 200 µm) mit n-Hexan und n-Hexan/Toluol 1:1 chromatographiert. In der ersten Fraktion wurden 202 mg (47 %) C₆₀ und in der zweiten Fraktion 96 mg (18 %; 34 % bezogen auf umgesetztes C₆₀) des in Beispiel 5 charakterisierten Monoadduktes erhalten.

### Beispiel 7

Eine Lösung von 36 mg (0.05 mmol) C₆₀ und 26 mg (0.15 mmol) p-Cyanobenzoylazid in 140 ml Dichlormethan wurde in Pyrexglasröhrchen gefüllt, mit Argon gespült und bei λₘₐₓ = 300 nm 40 Minuten im RPR 100 Rayonet Photochemical Chamber Reactor mit RPR 3000-Å Lampen bestrahlt. Dabei änderte sich die Farbe der Lösung von violett nach braunrot. Das Lösungsmittel wurde im Vakuum bei 40°C abdestilliert und das Reaktionsgemisch durch Säulenchromatographie (200 g Merck Kieselgel 60, Korngröße 63 bis 200 µm; Toluol/n-Hexan 50:50) getrennt. Nach Entfernen des Lösungsmittels wurde 5 mg (12 %) der 6,6-verbrückten Verbindung der Formel 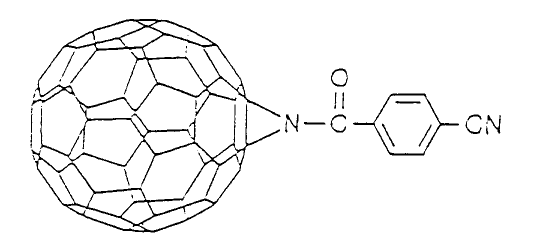
als mikrokristalliner schwarzgrauer Feststoff gewonnen.
MS m/z (%)
DEI (70 kV): 865 (19), 864 (27; MH^{·}⁺), 720 (100; M^{·}⁺-C₈H₄N₂O).
¹H-NMR (360 MHz; CS₂(Aceton-d₆ 10:1)
- δ (ppm):: 8.65 (m,2H), 8.03 (m,2H) ppm.
¹³C-NMR (90.5 MHz; CS₂/Aceton-d₆ 10:1)
- δ:: 168.91, 145.84, 145.75, 145.57, 145.45, 145.13, 144.99, 144.59, 144.36, 143.76 (2x), 143.75, 143.43, 142.78, 142.60, 141.76, 140.69, 134.89, 133.59, 130.31, 118.36, 117.54, 81.32 ppm.
FT-IR (KBr, 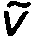 in cm⁻¹⁾
2228w, 1700s, 1696s, 1428m, 1400s, 1317w, 1296w, 1263ss, 1183w, 1093s, 1042s, 1016s, 950w, 862w, 802sh, 798ss, 727w, 691m, 578w, 573w, 565w, 555w, 544w, 526ss, 525sh.
UV-Vis λₘₐₓ in nm
(Toluol/n-Hexan 50:50):322, 425, 492.

### Beispiel 8

Das in Beispiel 7 charakterisierte Monoaddukt wurden ebenfalls folgendermaßen hergestellt.

Eine Lösung von 143 mg (0.20 mmol) C₆₀ und 137 mg (0.77 mmol) 4-Cyanobenzoylazid in 40 ml 1,1,2,2-Tetrachlorethan wurde 4.5 Stunden analog zu Beispiel 7 bestrahlt und analog zu Beispiel 2 aufgearbeitet. Der Extrakt wurde an Kieselgel (Korngröße 63 bis 200 µm) mit n-Hexan/Toluol 2:1 bis 1:1 chromatographiert. In der ersten Fraktion wurden 52 mg (36 %) C₆₀ und in der zweiten Fraktion 47 mg (27 %; 42 % bezogen auf umgesetztes C₆₀) des in Beispiel 7 charakterisierten Monoadduktes erhalten.

### Beispiel 9

Eine Lösung von 33.8 mg (0.04 mmol) des 6-6-Ring Fullerenaziridines aus Beispiel 1 in 40 ml 1,1,2,2-Tetrachlorethan wurde 18 Stunden refluxiert, wobei sich die zunächst weinrote Lösung zu einer braunroten Lösung veränderte. Durch Zugabe von Acetonitril wurde ein Niederschlag erhalten, der mit Acetonitril gewaschen und anschließend mit Trichlormethan eluiert wurde. Nach Entfernen des Lösungsmittels wurden 30.4 mg (90 %) der 6,6-verbrückten Verbindung der Formel 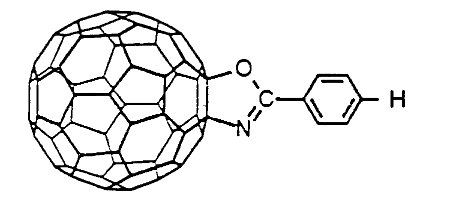
erhalten.
DCI-MS (NH₃), m/z (%)): 842 (30), 841 (68), 840 (100; MH⁺).
¹H-NMR (360 MHz; CS₂/Aceton-d₆ 10:1):
- δ =: 8.46 (m,2H), 7.71 (m,1H), 7.67 (m,2H) ppm.
¹³C-NMR (90.5 MHz; CS₂/Aceton-d₆ 10:1):
- δ =: 165.73, 148.76 (2x), 148.35, 146.95, 146.94, 146.81, 146.66, 146.61, 146.31, 146.18, 146.04, 145.80, 145.68, 145.20, 145.16, 144.85, 144.43, 144.30, 143.37, 143.35, 143.26, 142.95, 142.94, 142.86, 142.71, 142.58, 141.01, 140.21, 138.42, 136.73, 133.13, 129.86, 129.44, 127.60, 97.92, 92.93 ppm.
FT-IR (KBr):
1642s, 1511m, 1506sh, 1450w, 1326m, 1262s, 1181w, 1092s, 1026s, 983s, 933w, 804br, 773w, 690s, 660w, 576w, 563m, 527ss.
UV/Vis λₘₐₓ in nm (ε in l mol⁻¹ cm⁻¹)
in n-Hexan: 212 (60000), 255 (49000), 315 (16000);
in Toluol: 322 (28000).

### Beispiel 10

Eine Lösung von 14 mg (0.015 mmol) des 6-6-Ring Fullerenaziridines aus Beispiel 3 in 40 ml 1,1,2,2-Tetrachlorethan wurde 15.5 Stunden refluxiert. Nach Aufarbeitung wie in Beispiel 9 wurden 13.6 mg (97 %) der 6,6-verbrückten Verbindung der Formel 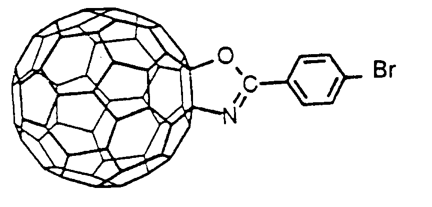
als braunes Pulver erhalten.
DCI-MS (NH₃), m/z (%)): 923 (5), 922 (24), 921 (63), 920 (100; MH⁺), 919 (75), 918 (70), 722 (14), 721 (63), 720 (100).
¹H-NMR (360 MHz; CS₂/Aceton-d₆ 10:1):
- δ =: 8.36 (m,2H), 7.82 (m,2H) ppm.
¹³C-NMR (90.5 MHz; CS₂/Aceton-d₆ 10:1):
- δ =: 165.15, 148.79, 148.47, 148.38, 146.98 (2x), 146.85, 146.70, 146.64, 146.35, 146.12, 146.02, 145.83, 145.71, 145.36, 145.16, 144.86, 144.03, 143.41, 143.37, 143.30, 142.95, 142.93, 142.87, 142.73, 142.59, 142.56, 141.05, 140.25, 138.42, 136.75, 132.82, 131.36, 128.47, 126.59, 98.15, 92.88 ppm.
FT-IR (KBr):
1643s, 1632w, 1591w, 1485m, 1398m, 1384w, 1322m, 1261s, 1190w, 1181w, 1089s, 1012s, 983s, 931m, 798br, 723m, 657w, 603w, 563w, 527ss.
UV/Vis λₘₐₓ in nm (ε in l mol⁻¹ cm⁻¹)
in n-Hexan: 210 (63000), 256 (36000), 315 (12000);
in Toluol: 320 (31000).

### Beispiel 11

Eine Lösung von 17.4 mg (0.02 mmol) des 6-6-Ring Fullerenaziridines aus Beispiel 5 in 30 ml 1,1,2,2-Tetrachlorethan wurde 18 Stunden refluxiert. Nach Zugabe von 200 ml Acetonitril wurde das Gemisch gekühlt und anschließend filtriert. Der Filterrückstand wurde mit Toluol eluiert, und nach Entfernen des Lösungsmittels wurden 16 mg (92 %) der 6,6-verbrückten Verbindung der Formel 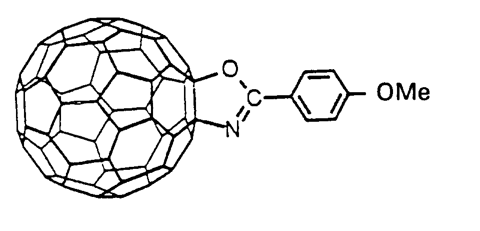
erhalten.
DCI-MS (NH₃), m/z (%)): 872 (9), 871 (24), 871 (51; MH⁺), 720 (100).
¹H-NMR (360 MHz; CS₂/Aceton-d₆ 10:1):
- δ =: 8.37 (m,2H), 7.15 (m,2H), 3.99 (s,3H) ppm.
¹³C-NMR (90.5 MHz; CS₂/Aceton-d₆ 10:1):
- δ =: 165.41, 163.66, 149.10, 148.73, 148.32, 146.92, 146.89, 146.77, 146.63, 146.57, 146.25, 146.18, 146.04, 145.76, 145.64, 145.43, 145.16, 144.84, 144.57, 143.33, 143.32, 143.22, 142.98, 142.91, 142.82, 142.67, 142.58, 142.55, 140.96, 140.14, 138.41, 136.64, 131.64, 119.75, 114.83, 97.76, 92.95, 55.78 ppm.
FT-IR (KBr):
1639s, 1607m, 1511s, 1420w, 1320br, 1172m, 1140w, 1088m, 1028m, 983s, 931m, 837m, 727m, 604w, 578w, 563m, 527ss.
UV/Vis λₘₐₓ in nm (ε in l mol⁻¹ cm⁻¹)
in n-Hexan: 210 (83000), 257 (72000), 314 (25000);
in Toluol: 322 (33000).

### Beispiel 12

Eine Lösung von 22,6 mg (0.026 mmol) des 6-6-Ring Fullerenaziridines aus Beispiel 7 in 30 ml 1,1,2,2-Tetrachlorethan wurde 3 Stunden refluxiert. Nach Aufarbeitung wie in Beispiel 9 wurden 20.5 mg (90 %) der 6,6-verbrückten Verbindung der Formel 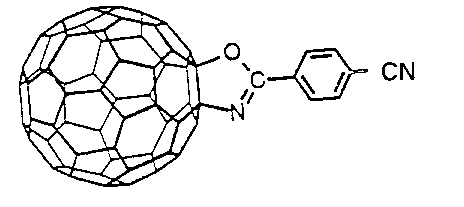
als braunes Pulver erhalten.
DCI-MS (NH₃), m/z (%)): 868 (5), 867 (31), 866 (72), 865 (100, MH⁺), 721 (5), 720 (10).
¹H-NMR (360 MHz; CS₂/Aceton-d₆ 10:1):
- δ =: 8.63 (m,2H), 8.01 (m,2H) ppm.
¹³C-NMR (90.5 MHz; CS₂/Aceton-d₆ 10:1):
- δ: = 164.64, 148.80, 148.39, 148.04, 147.01, 147.00, 146.87, 146.71, 146.65, 146.39, 146.06, 145.97, 145.84, 145.73, 145.27, 145.13, 144.84, 144.42, 143.43, 143.38, 143.32, 142.95, 142.87 (2x), 142.73, 142.59, 142.50, 141.08, 140.28, 138.39, 136.77, 133.13, 131.35, 130.31, 117.81, 117.15, 98.38, 92.79 ppm.
FT-IR (KBr):
2230w, 1642s, 1408w, 1326m, 1262s, 1092s, 1020s, 982m, 929w, 849w, 803br, 658w, 603w, 577w, 563m, 527s, 525sh.
UV/Vis λₘₐₓ in nm (ε in l mol⁻¹ cm⁻¹)
in n-Hexan: 210 (62000), 256 (50000), 314 (17000);
in Toluol: 321 (37000).

### Beispiel 13

Zu einer Lösung von 60 mg (0.082 mmol) C₆₀ in 25 ml Toluol wurden 40 mg (0.176 mmol) N-Chloramin T und 2 mg Tetrabutylammoniumchlorid als Phasentransferkatalysator gegeben. Es wurde bei 25°C gerührt. Nach 7 Tagen wurde das Reaktionsgemisch filtriert, die Lösung auf die Hälfte eingeengt und an Kieselgel chromatographiert. (SiO₂, Korngröße 63 bis 200 µm, Toluol/iso-Hexan 1:2 bis reines Toluol). Neben nicht umgesetzten C₆₀ wurden 20 mg (≙ 27 % der theoretischen Ausbeute) der 6,6-verbrückten Verbindung der Formel 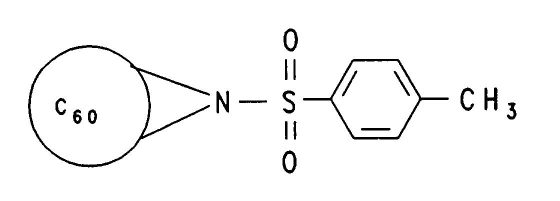
isoliert.
R_{f} (SiO₂/Toluol) = 0.57
MS (FAB) = 889 (M^{⊖}, 10 %) 720 (100 %)
¹H-NMR (360 MHz, CS₂/CDCl₃):
- δ =: 8.19 (m, 2H), 7.51 (d, 2H), 2.58 (s, 3H).
¹³C-NMR (75 MHz, CS₂/CDCl₃):
- δ =: 145.17 (2C), 145.10, 144.93, 144.85, 144.78 (2C), 144.32, 143.97, 143.79 (2C), 143.71, 143.13, 142.96, 142.90, 142.59 (1C), 141.99, 141.68, 141.16, 140.72, 135.76 (1C), 129.90 (2C), 128.39 (2C), 79.70 (2C), 21.76 (1C).

## Patentansprüche

1. Fullerenderivat der Formel I 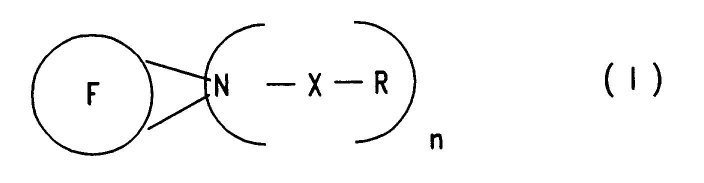 worin die Symbole und Indices folgende Bedeutung haben:
F: ein Fullerenrest der Formel C₂₀₊₂ₘ mit m = 2 bis 100;
X: C = O, SO₂, SO, P(O)R, P(S)R oder PR;
R: eine geradkettige oder verzweigte C₁-C₂₀-Alkylgruppe, eine
C₆-C₁₄-Aryl-C₁-C₁₀-alkylgruppe oder eine C₆-C₁₄-Arylgruppe, wobei in der Alkylgruppe eine oder mehrere nicht benachbarte CH₂-Gruppen durch -C ≡ C-, -CH = CH-, -O-, -S-, -COO-, -SiR₂- und/oder -CO- ersetzt sein können, Alkyl und Aryl unabhängig voneinander ein- oder mehrfach gleich oder verschieden durch OH, OR¹, COOR, OCOR, F, Cl, Br, NO₂, CN, NHCOR¹ oder NR¹COR¹ sowie Aryl durch R¹ substituiert sein können und R¹ eine C₁-C₆-Alkylgruppe ist;
n: eine natürliche Zahl von 1 bis 10 + m mit m = 2 bis 100.

2. Fullerenderivat nach Anspruch 1, dadurch gekennzeichnet, daß
F: ein Fullerenrest der Formel C₂₀₊₂ₘ mit m = 20, 25, 28 und/oder 29;
X: C = O, SO₂ oder SO,
R: die obengenannte Bedeutung hat, und
n: eine natürliche Zahl von 1 bis 10 + m mit m = 20, 25, 28 und/oder 29 ist.

3. Fullerenderivat nach Anspruch 1, dadurch gekennzeichnet, daß
F: C₆₀ und/oder C₇₀;
X: C = O oder SO₂;
R: eine geradkettige oder verzweigte C₁-C₁₀-Alkylgruppe, eine Phenyl-C₁-C₆-alkylgruppe oder eine Phenylgruppe, wobei Alkyl und Phenyl unabhängig voneinander ein- bis dreifach gleich oder verschieden durch OH, OR¹, COOR, OCOR, F, Cl, Br, NO₂, CN, NHCOR¹ oder NR¹COR¹, sowie Phenyl durch R¹ substituiert sein können und R¹ eine C₁-C₆-Alkylgruppe ist, und
n: eine natürliche Zahl von 1 bis 6 ist.

4. Fullerenderivat nach Anspruch 1, dadurch gekennzeichnet, daß
F: C₆₀;
X: C = O oder SO₂;
R: eine geradkettige oder verzweigte C₁-C₆-Alkylgruppe oder eine Phenylgruppe, wobei Alkyl und Phenyl unabhängig voneinander ein-bis dreifach gleich oder verschieden durch OH, OR², COOR², OCOR², F, Cl, Br, NO₂ oder CN sowie Phenyl durch R² substituiert sein können und R² Methyl oder Ethyl ist, und
n: 1 oder 2 ist.

5. Verfahren zur Herstellung eines Fullerenderivats der Formel I gemäß Anspruch 1, dadurch gekennzeichnet, daß ein Fulleren der Formel C₂₀₊₂ₘ, worin m = 2 bis 100 ist, mit einem Säureazid der Formel II
R-X-N₃ (II),
wobei R und X die oben genannten Bedeutungen haben, in einem inerten Lösungsmittel bei einer Temperatur von -78°C bis 180°C zu einer Verbindung der Formel I umgesetzt wird.

6. Verfahren gemäß Anspruch 5, dadurch gekennzeichnet, daß die Umsetzung in Gegenwart einer Lichtquelle bei einer Wellenlänge von 230 bis 380 nm erfolgt.

7. Verfahren gemäß Anspruch 5 oder 6, dadurch gekennzeichnet, daß als inertes Lösungsmittel 1,1,2,2-Tetrachlorethan eingesetzt wird.

8. Verfahren zur Herstellung eines Fullerenderivats der Formel I gemäß Anspruch 1, dadurch gekennzeichnet, daß ein Fulleren der Formel C₂₀₊₂ₘ, worin mit m = 2 bis 100 ist, mit einem stabilisierten Stickstoffanion der Formel III 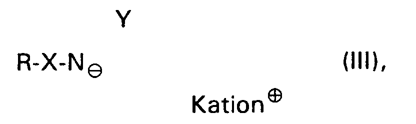 wobei R und X die oben genannten Bedeutung haben,
Y: -Cl, -Br oder -I und
Kation: Alkalimetall, Tetraalkyl(aryl)ammonium, Tetraalkyl(aryl)phosphonium oder Hexaalkyl(aryl)guanidinium
bedeuten, in einem aprotischen Lösungsmittel bei einer Temperatur von -78°C bis 180°C zu einer Verbindung der Formel I umgesetzt wird.

9. Fullerenderivat der Formel IV 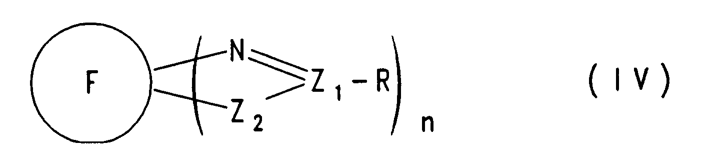 worin die Symbole und Indices folgende Bedeutung haben:
F: ein Fullerenrest der Formel C₂₀₊₂ₘ mit m = 2 bis 100;
Z₁: C, S, S = O oder P;
Z₂: O oder S;
R: eine geradkettige oder verzweigte C₁-C₂₀-Alkylgruppe, eine C₆-C₁₄-Aryl-C₁-C₁₀-alkylgruppe oder eine C₆-C₁₄-Arylgruppe, wobei in der Alkylgruppe eine oder mehrere nicht benachbarte CH₂-Gruppen durch -C ≡ C-, -CH = CH-, -O-, -S-, -COO-, -SiR₂- und/oder - CO-ersetzt sein können, Alkyl und Aryl unabhängig voneinander ein- oder mehrfach gleich oder verschieden durch OH, OR¹, COOR, OCOR, F, Cl, Br, NO₂, CN, NHCOR¹ oder NR¹ COR¹ sowie Aryl durch R¹ substituiert sein können und R¹ eine C₁-C₆-Alkylgruppe ist;
n: eine natürliche Zahl von 1 bis 10 + m mit m = 2 bis 100.

10. Verwendung der Fullerenderivate der Formel I gemäß Anspruch 1 und der Formel IV gemäß Anspruch 9 in optoelektrischen Bauelementen.
